# FASCICULE DE BREVET EUROPEEN

(11) **EP 2 519 294 B1**
(45) Date de publication et mention de la délivrance du brevet: **22.11.2017**
(21) Numéro de dépôt: 10810785.5
(22) Date de dépôt: 23.12.2010
(51) Int. Cl.: A61M 11/02, A61M 15/08, A61M 15/00, A61M 11/08, A61M 16/00, A61M 11/00

(54) **DISPOSITIF D'ADMINISTRATION D'AÉROSOL PAR VOIE BUCCALE À VISÉE RHINOPHARYNGÉE, NASALE OU NASOSINUSIENNE**
VORRICHTUNG ZUR ORALEN VERABREICHUNG EINES AEROSOLS FÜR DEN RHINOPHARYNX, DIE NASENHÖHLEN ODER DIE NASENNEBENHÖHLEN
DEVICE FOR ORAL ADMINISTRATION OF AN AEROSOL FOR THE RHINOPHARYNX, THE NASAL CAVITIES OR THE PARANASAL SINUSES

(30) Priorité: 28.12.2009 FR 0959622
(43) Date de publication de la demande: 07.11.2012
(73) Titulaire: LA DIFFUSION TECHNIQUE FRANCAISE, 42000 Saint Etienne (FR); Université François-Rabelais, 37000 Tours (FR)
(72) Inventeur: VECELLIO-NONE, Laurent, F-31170 Chambray Les Tours (FR); CHANTREL, Gilles, F-42100 Saint Etienne (FR); MASSARDIER, Michel, F-42000 Saint-etienne (FR)
(74) Mandataire: Cabinet Laurent & Charras
(86) Numéro de dépôt international: PCT/FR2010/052896
(87) Numéro de publication internationale: WO 2011/080473

(56) Documents cités:
- WO-A1-02/13896
- WO-A1-98/53869
- WO-A1-2005/120617
- WO-A2-02/068031
- WO-A2-2004/103447
- US-B1- 6 752 147

## Description

L'invention se rattache au secteur technique des systèmes de génération d'aérosols et de sprays à visées médicales.

Les aérosols sont définis comme une suspension de particules dans un gaz. Ces particules peuvent avoir une taille allant de quelques nanomètres à plusieurs dizaines de micromètres. Les systèmes de génération d'aérosols médicaux ont pour fonction de transformer, un liquide ou une poudre, médicamenteux sous forme d'aérosol pour être administré dans les voies respiratoires.

L'avantage de la voie aérosol en comparaison des autres voies d'administration est le ciblage de l'organe à traiter par dépôt du médicament. Les nébuliseurs actuels permettent d'administrer de grandes quantités de médicaments dans les voies respiratoires. Les nébuliseurs à visée pulmonaire ont pour cible les poumons, les nébuliseurs ou sprays à visée nasale ont pour cible les fosses nasales et le rhinopharynx. Dans le cadre des nébuliseurs ou des sprays à visée nasale, il est théoriquement possible de déposer l'aérosol uniquement dans les fosses nasales, lieu du premier passage de l'aérosol dans les voies aériennes. Une première solution est d'utiliser un aérosol avec une taille de particules importantes. Le problème avec un aérosol de forte granulométrie est qu'il ne se déposera pas de façon périphérique et homogène dans les différents compartiments de la sphère ORL (exemples : sinus, site cible pour traiter les sinusites) (Suman et al, Pharm Res. 1999, 6:1648-52). L'autre solution consiste en l'utilisation d'un aérosol de faible granulométrie pour tenter d'assurer un dépôt « périphérique » dans la sphère ORL. En revanche, cet aérosol fin est susceptible de se déposer dans les poumons. D'autre part, compte tenu de l'anatomie des narines et du rhinopharynx, les aérosols pénétrant dans les narines vont être filtrés par les poils du nez et subir une forte accélération due au faible diamètre des narines et de la valve nasale (C Croce et al, Ann Biomed Eng. 2006, 34:997-1007). Les particules ainsi véhiculées au-delà des premiers centimètres des fosses nasales auront une faible taille incompatible avec un dépôt par impaction ou sédimentation dans le rhinopharynx ou les sinus. Selon une étude réalisée avec un modèle de tête inhalant un aérosol d'un MMAD de 5µm, 82% de l'aérosol est déposé dans le nez, la valve nasale et les premiers centimètres de fosses nasales, 0.2% dans les sinus, 1% dans le reste des fosses nasales et 26.8% dans les poumons (Vecellio, 2002, thèse de doctorat). Ainsi, selon cette étude par imagerie scintigraphique dans un modèle de tête plastinée, seul 5% de l'aérosol ayant traversé la valve nasale s'y est déposé.

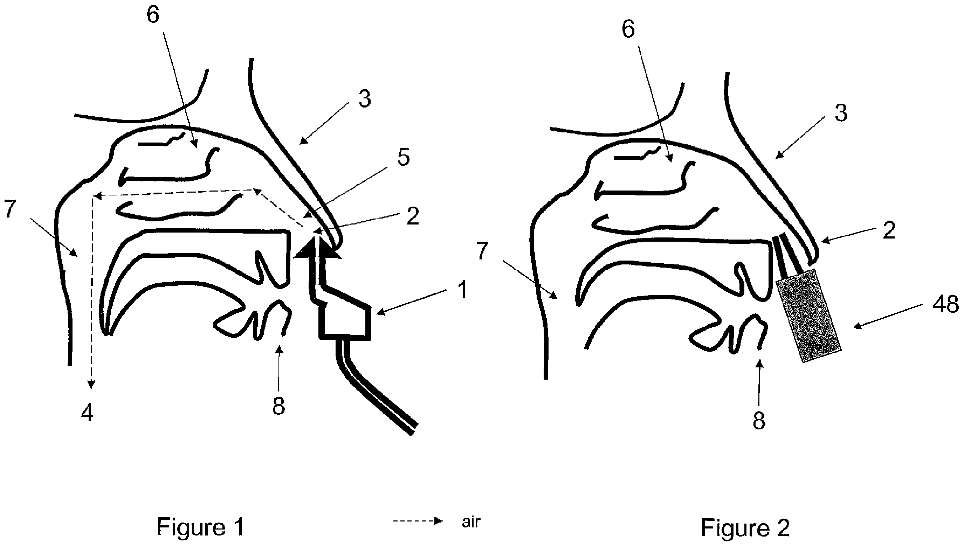

Dans le présent texte, les voies aériennes supérieures nasales peuvent être décrites comme la succession des régions anatomiques suivantes (Figure 1): les narines (2), la valve nasale (5), les fosses nasales (6) et le rhinopharynx (7). Les fosses nasales représentant le volume anatomique le plus important et incluent la région de l'ethmoïde, des cornets et l'accès au sinus.

Le nébuliseur pneumatique Atomisor NL11 (FR2835435) pose ce problème de ciblage des fosses nasales (Figure 1). Dans son principe d'utilisation, le nébuliseur pneumatique Atomisor NL11 (1) muni de son embout nasal (FR2638361) est connecté aux deux narines (2), droite et gauche et génère un aérosol de 5µm dans la sphère ORL (3) du patient durant la phase inspiratoire (Figure 1). Durant la phase inspiratoire (Figure 1), l'aérosol produit par le nébuliseur (1) est alors directement dirigé depuis la sphère ORL (3) vers les poumons du patient (4). L'aérosol produit est alors accéléré dans les premiers centimètres des fosses nasales et encore davantage au niveau de la valve nasale (5), expliquant sa forte impaction dans les premiers centimètres des narines. De plus, les poils, premier élément naturel de protection des voies respiratoires par filtration, interceptent les particules les plus grosses. Les particules les plus fines ayant traversées la valve nasale se retrouvent au niveau des fosses nasales (6), lesquelles disposent d'une anatomie moins propice au dépôt de particules par impaction que celle des narines (vitesses d'air moins importantes que dans les narines).

Les sprays nasaux (48) utilisent un embout narinaire d'une longueur suffisante pour assurer le passage du dispositif à travers les poils (figure 2). Ce type de dispositif produit des particules de large taille (20µm à 150µm) avec de fortes vitesses de particules initiales assurant leur dépôt par impaction. L'angle du spray est alors un paramètre important pour assurer une homogénéité de dépôt dans le rhinopharynx. Comme décrit dans la littérature (Kimbell et al, 2007, J Aerosol Med, 20 :59-74), ce type de dispositif d'administration par spray trouve ses limites dans la variabilité d'utilisation. En effet, l'emplacement, l'angle d'orientation de l'embout nasal du dispositif conditionnent le dépôt de particules et donc l'efficacité du traitement. D'autre part, compte tenu de la taille des particules et de la vitesse d'injection des particules, les particules sembleraient n'atteindre que très faiblement les fosses nasales médianes (Senocak et al, 2005, Otolaryngology Haed and Neck Surgery, 133 :944-948) et ne pas atteindre les fosses nasales postérieures (Cheng et al, 2001, J Aerosol Med, 14 :267-280) (Guo et al, 2005, Pharm R, 22 :1871-1878).

Pour palier à ce problème de ciblage d'aérosol fin dans la sphère ORL, différents systèmes commercialisés proposent des solutions plus ou moins efficaces.

Le nébuliseur Pari sinus met en oeuvre les brevets (US2006/0162722 A1, US2007/0181133 A1). Il administre l'aérosol fin par une narine, au moment où le sujet ferme le voile du palais, pour limiter le dépôt dans les poumons et augmenter le dépôt dans la sphère ORL. L'aérosol pénètre dans une narine et ressort par l'autre narine munie d'un second embout narinaire avec rétrécissement de section pour augmenter la pression nasale et favoriser la pénétration d'aérosol dans les sinus. Ce mode d'administration de l'aérosol demande une participation active du patient. Le patient ne doit ni inspirer ni expirer durant l'administration de l'aérosol et doit simultanément relever le voile du palais. Ce système demandant une participation très active du patient peut se révéler inefficace si le patient n'exécute pas correctement les consignes pour relever le voile du palais. Cela nécessite l'éducation et la formation du patient, ce qui n'est pas toujours réalisable selon les contraintes d'âge de ceux-ci. Ce système ne permet pas de s'affranchir du fort dépôt dans les premiers centimètres des narines.

Le système Optinose mettant en oeuvre les brevets (WO 03/000310 A2, EP1410820A2, US 2006/0107957 A1, US2005/035992 A1, US 2006/0096589 A1) utilise également le système de pénétration de l'aérosol dans une des deux narines et son échappement par l'autre narine. Il utilise en plus un déclenchement automatique de la génération de l'aérosol durant la phase expiratoire buccale du patient. Dans ces conditions, lors de la phase inspiratoire, le patient peut inspirer par la narine et inhaler de l'air dépourvu d'aérosol. Lors de la phase expiratoire buccale, le voile du palais est remonté, et l'aérosol produit pénètre dans une des deux narines.

L'aérosol est alors transporté de la première narine vers la deuxième narine et les poumons sont protégés de toute pénétration d'aérosol par l'étanchéité du voile du palais. La performance de ce système pour limiter le dépôt pulmonaire a été démontrée sur des sujets sains mais l'aérosol ne pénètre pas par un embout buccal mais toujours par un embout nasal. (Djupesland et al, Bi-directional nasal delivery of aerosols can prevent lung deposition. J Aerosol Med. 2004 Fall;17(3):249-59). Le brevet WO2007093784 de la même société décrit également un système de génération d'aérosol uniquement durant la phase expiratoire nasale. L'inconvénient de ce système est que l'aérosol pénètre par une narine et non par la bouche ainsi il ne permet pas de s'affranchir du fort dépôt d'aérosol dans les premiers centimètres des narines.

Selon l'état de l'art antérieur, force est de constater que l'administration d'aérosol à visée rhinopharyngée, nasale ou nasosinusienne est toujours réalisée au moyen d'un embout nasal introduit dans les narines. Cette voie d'administration nasale est la conséquence logique des études démontrant l'intérêt de l'utilisation d'un embout buccal pour favoriser le dépôt pulmonaire. En effet, l'utilisation d'un masque facial chez le patient permet à la fois l'inhalation de l'aérosol par la bouche mais également par le nez limitant ainsi le dépôt pulmonaire et favorisant le dépôt rhinopharyngé. L'utilisation d'un embout buccal est donc recommandée pour l'administration d'aérosol à visée pulmonaire (Dautzenberg B, Becquemin MH, Chaumuzeau JP, Diot P. 2007. Bonnes pratiques de l'aérosolthérapie par nébulisation. Rev Mal Respir. 24 :751-757) et l'embout nasal est recommandé pour l'administration d'aérosol à visée rhinopharyngée. En résumé, l'administration de l'aérosol à visée pulmonaire est réalisée via la bouche ou le nez du patient et l'administration de l'aérosol à visée nasale est réalisée via le nez du patient (Tableau 1).

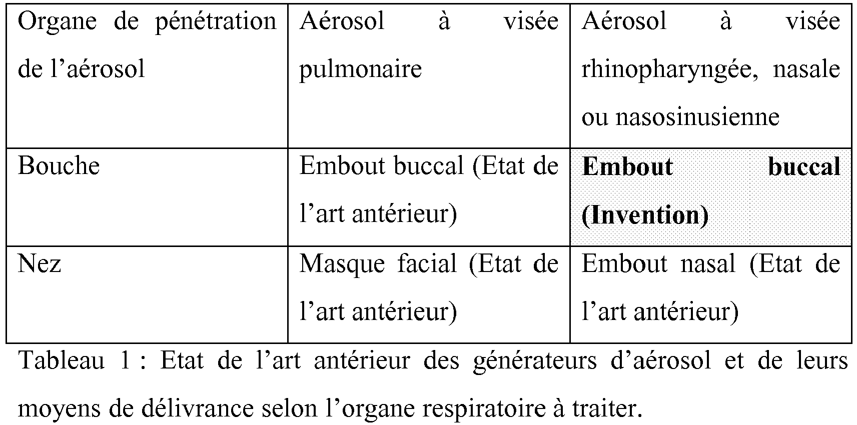

On connaît par ailleurs par le brevet WO 2004/103447 un dispositif muni d'un embout buccal et d'un tuyau d'administration pénétrant profondément dans la cavité orale afin d'assurer le dépôt de la substance par spray sur la muqueuse ou la cavité orale avec ainsi une projection ciblée ayant un effet limité à un endroit donné.

On connaît aussi par le brevet WO 98/533869 une méthode pour introduire une substance dans le nez d'une personne par l'utilisation d'un dispositif tubulaire sous forme d'une paille insérée en sa première ouverture dans la bouche du patient et en sa seconde ouverture dans la narine du patient. Le patient expire par la bouche dans le dispositif tubulaire afin de transférer la substance dans la narine. Lors de la phase expiratoire buccale le voile du palais est relevé, rendant étanche la communication entre la cavité buccale et la cavité nasale. La substance peut pénétrer dans la cavité nasale sans risque de dépôt dans la cavité buccale ou les poumons du patient. En sens inverse, la méthode est physiquement impossible.

Ces documents ont donc des applications et effets très limités.

La démarche du Demandeur a donc été de reconsidérer le problème de ce ciblage de la sphère ORL avec un aérosol.
Face à cette situation, le Demandeur s'est alors orienté sur une conception différente de ce type d'appareils.

Selon une première caractéristique, le système de génération d'aérosol est remarquable en ce qu'il est constitué d'un générateur d'aérosol délivrant l'aérosol au moyen d'un embout buccal au niveau de la bouche du patient pour traiter le rhinopharynx, les sinus, les fosses nasales et les narines (Tableau 1).
Le terme embout buccal utilisé ici recouvre un embout ou raccord pénétrant dans la bouche aussi bien qu'un masque buccal appliqué sur la bouche.

Selon une autre caractéristique, le dispositif d'administration d'aérosol constitué d'un générateur de particules dont la taille est comprise entre 10 nm et 200 µm, d'un embout buccal ou masque buccal permettant l'administration de l'aérosol par la bouche durant la phase d'expiration nasale ou durant la phase de pause respiratoire précédent l'expiration nasale et d'une source de gaz ou pression pour véhiculer les particules, est remarquable en ce que l'embout buccal est étanche à l'air et pénètre au-delà des dents du patient selon une longueur de 4 cm maximale et constitue le moyen d'administration de l'aérosol à visée nasale, rhinopharyngée ou nasosinusienne durant l'administration de l'aérosol et permettant d'acheminer l'aérosol successivement dans la bouche, le rhinopharynx puis les fosses nasales et les sinus puis son échappement par une ou les deux narines du patient, et en ce que le dispositif n'autorise pas l'expiration buccale lors des phases d'administration de l'aérosol, les particules d'aérosols n'étant pas dirigées vers les poumons.

Ces caractéristiques et d'autres encore ressortiront bien de la suite de la description.
- La figure 1 illustre l'état de l'art antérieur pour la délivrance nasale avec un nébuliseur.
- La figure 2 illustre l'état de l'art antérieur pour la délivrance nasale avec un spray.
- Les figures 3, 4, 5 et 6 illustrent le principe de la méthode de l'administration de l'aérosol permettant l'inspiration et l'expiration par la bouche hors des phases d'administration de l'aérosol
- Les figures 7, 8, 9 et 10 illustrent le principe de la méthode de l'administration de l'aérosol avec un dispositif de type spray actionné manuellement et ne permettant ni l'expiration ni l'inspiration par la bouche.
- Les figures 11, 12, 13 et 14 illustrent le principe de la méthode de l'administration de l'aérosol avec un dispositif de type spray valvé actionné manuellement ne permettant pas l'expiration par la bouche.
- Les figures 15 et 16 illustrent le principe de fonctionnement du système avec un générateur de type poudre et un réservoir de gaz externe pour la délivrance manuelle de l'aérosol.
- Les figures 17 et 18 illustrent le principe de fonctionnement du système selon l'invention avec générateur de type flacon pressurisé et un réservoir de gaz externe pour la délivrance automatique de l'aérosol.
- Les figures 19 et 20 illustrent le principe de fonctionnement du système dans son application avec un nébuliseur pneumatique et un moyen automatique d'administration des particules.
- Les figures 21 et 22 illustrent le principe de fonctionnement du système dans son application avec un nébuliseur à tamis et une chambre de stockage associés à un moyen automatique d'administration des particules.
- Les figures 23, 24, 25 et 26 illustrent le principe de fonctionnement du système dans son application avec un nébuliseur pneumatique associé à une onde acoustique et à un moyen automatique d'administration des particules.
- Les figures 27, 28, 29 et 30 illustrent le principe de fonctionnement du système dans son application avec un réservoir de gaz externe pour la délivrance automatique de l'aérosol associé à un embout nasal créant une surpression dans les fosses nasales pour créer la pénétration de l'aérosol dans les sinus maxillaires.
- Les figures 31, 32, 33 et 34 illustrent le principe de fonctionnement du système avec un générateur de poudre et un réservoir de gaz externe pour la délivrance manuelle de l'aérosol durant la première partie de la pause inspiratoire.
- La figure 35 représente l'imagerie scintigraphique du dépôt de l'aérosol selon l'invention
- La figure 36 représente l'imagerie scintigraphique du dépôt de l'aérosol avec un nébuliseur délivrant l'aérosol au moyen d'un embout nasal.

La respiration du patient peut être décomposée en différentes phases : La phase inspiratoire correspondante à la pénétration de l'air extérieur dans les poumons du patient, la pause inspiratoire correspondante à une pause de la respiration du patient à la fin de son inspiration, la phase expiratoire correspondante à l'évacuation de l'air contenu dans ses poumons vers l'extérieur du patient et la pause expiratoire correspondante à une pause de la respiration du patient à la fin de son expiration. L'invention concerne une méthode de délivrance d'aérosol dans la bouche du patient au moyen d'un embout buccal pour le ciblage et le traitement du rhinopharynx, des sinus, des fosses nasales et du nez. L'invention concerne également un système d'administration d'aérosol délivrant l'aérosol au moyen d'un embout buccal dans la bouche du patient lors de sa phase expiratoire nasale ou lors des phases de pauses respiratoires. Cette administration peut être réalisée durant la totalité ou durant la première partie de ces phases. L'invention est donc une méthode et des moyens d'administration d'un aérosol nasal via la seconde ouverture des organes respiratoires avec l'air ambiant qu'est la bouche. Le poumon (4) est une structure déformable, assurant la pénétration de l'air via la bouche (8) ou le nez (2) par ses modifications de volumes. Pour atteindre le poumon à l'aide d'un aérosol, il est nécessaire de passer par la trachée. Il n'existe qu'une ouverture dans le poumon pour y pénétrer. Dans le cas de la sphère ORL, la situation est différente. La sphère ORL est une structure pouvant être considérée comme non déformable et possédant deux ouvertures en contact avec l'air ambiant. Il est donc théoriquement possible de faire pénétrer l'aérosol par une ouverture ou par l'autre ouverture. La première ouverture est constituée par les narines (2) et pose les problèmes de dépôt d'aérosol précédemment décrits. La deuxième ouverture est la bouche (8) et est traditionnellement utilisée pour l'administration de l'aérosol à visée pulmonaire. La comparaison anatomique de ces deux ouvertures montre l'intérêt du passage de l'aérosol par la bouche (8) (en passant par le rhinopharynx (7)) pour assurer la pénétration des plus grosses particules dans les fosses nasales (6).

Les systèmes de génération d'aérosol sont classiquement dissociés selon deux grandes catégories, les nébuliseurs et les aérosols doseurs. Les nébuliseurs sont des dispositifs générant de grandes quantités de liquide sous forme aérosol. Ils demandent une préparation préalable par introduction du médicament dans le réservoir du nébuliseur et sont utilisés pour des patients dont la pathologie est sévère. Par opposition aux nébuliseurs, les aérosols doseurs sont des dispositifs délivrant de petites quantités d'aérosols calibrées. Ces derniers peuvent être à base de poudre (aérosol doseur de poudre) ou de liquide (sprays) et offrent l'avantage d'être portables et souvent pré conditionnés avec le médicament. Ces aérosols doseurs sont utilisés dans le cadre de patients dont la pathologie est stable.

Pour la description de la présente invention, nous choisirons de dissocier les générateurs d'aérosol selon qu'ils utilisent ou non un gaz ou un liquide sous pression pour générer l'aérosol. Ainsi, les nébuliseurs pneumatiques et les aérosols doseurs pressurisés sont des générateurs d'aérosols utilisant un gaz sous pression. De même certains sprays sont également produits à l'aide d'une surpression de liquide. En revanche, les aérosols doseurs de poudre passifs, les nébuliseurs à tamis (ou à membrane) et les nébuliseurs ultrasoniques sont des générateurs d'aérosol ne nécessitant pas de gaz ni de liquide sous pression pour générer l'aérosol.

Le déplacement des particules constituant l'aérosol peut être effectué au moyen du gaz vecteur de l'aérosol. Les particules peuvent également être mises en mouvement de part leur éjection initiale lors de leur phase de génération sous pression. Cette vitesse initiale non nulle créée un déplacement de la particule depuis le générateur vers la bouche du patient. Ainsi le transport des particules depuis le générateur vers la bouche du patient peut être réalisé par le générateur lui-même (vitesse initiale de la particule) ou par le gaz vecteur. Le mouvement du gaz peut être réalisé par un moyen mécanique comme par exemple un ventilateur, un compresseur ou encore une « poire » à action manuelle (structure déformable). Le mouvement de la particule peut être réalisé au moyen d'un liquide sous pression (seringue par exemple). L'administration de l'aérosol dans la bouche du patient lors des phases de pauses respiratoires ou lors des phases expiratoires nasales peut être réalisée par le patient lui-même (par exemple, déclenchement manuel) ou de façon automatique par le système. L'automatisation peut être réalisée au moyen d'un capteur (de pression ou de débit par exemple) ou bien encore à l'aide d'un moyen mécanique (brevet n° 9313478 du présent demandeur par exemple). Le capteur peut être placé sur le circuit relié à la bouche ou sur le circuit relié aux narines du patient. Dans le cas d'un générateur d'aérosol fonctionnant au moyen d'un gaz source, le système d'automatisation déclenchera la génération du gaz source lors des phases expiratoires nasales du patient ou encore lors des phases de pauses respiratoires. L'aérosol sera alors à la fois généré et transporté par le gaz source.

Le système pourra également générer l'aérosol, en continu ou non, dans une chambre de stockage, d'où il ne sera transporté vers le patient que lors des phases expiratoires nasales ou lors des phases de pauses respiratoires.

Dans le cas d'un générateur d'aérosol ne nécessitant pas l'utilisation de gaz source pour la génération de particules, le système déclenchera la génération des particules et un mouvement de gaz pour transporter les particules vers la bouche du patient.

L'administration buccale de l'aérosol ne sera pas réalisée durant la phase inspiratoire du patient. Ceci est assuré à l'aide d'un moyen automatique administrant l'aérosol durant la phase expiratoire nasale ou durant la pause respiratoire du patient. Ainsi, l'efficacité du traitement n'est pas fonction de la bonne exécution de l'administration de l'aérosol par le patient durant sa phase respiratoire. Le système d'administration peut être également clos et étanche pour ne pas autoriser l'expiration du patient par la bouche, mais uniquement l'expiration par le nez.

Un dispositif illustrant le principe de à l'invention (mais non conforme à l'invention) peut être représenté dans sa configuration la plus simple par un dispositif comprenant un générateur de particules dont la taille est comprise entre 10 nm et 200µm et un embout buccal.

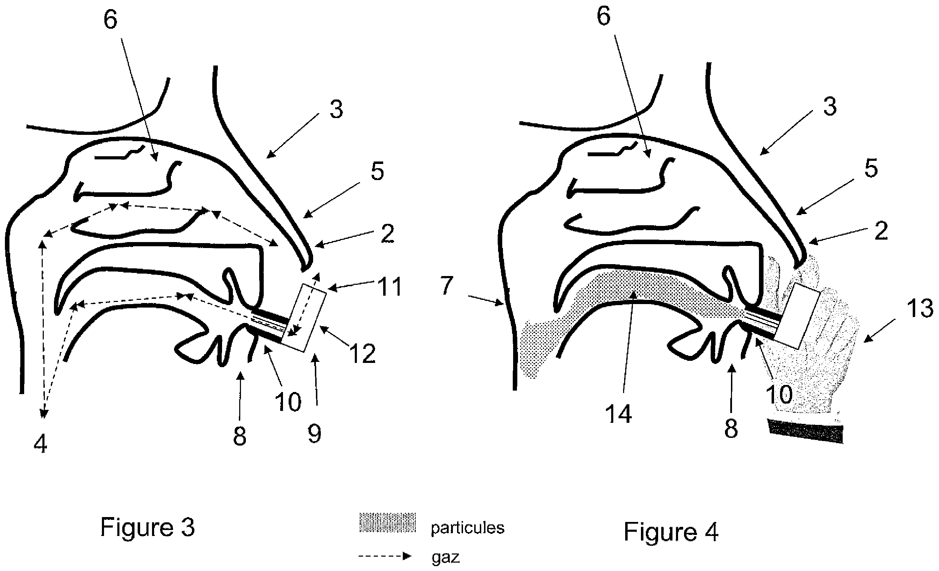

Ainsi dans une mise en oeuvre simple (Figure 3), le dispositif (9) est un circuit ouvert relié à la bouche (8) du patient comprenant un embout buccal (10) muni d'une ouverture (11) avec l'air ambiant et relié à un générateur (12) délivrant des particules avec une vitesse initiale et fonctionnant sans addition de gaz.

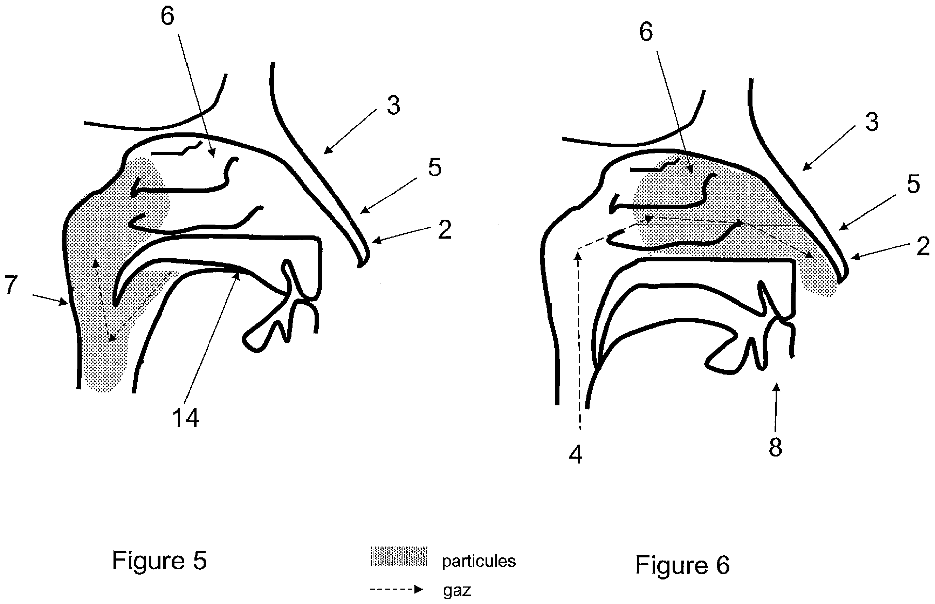

Ainsi, le système (9) est un circuit ouvert permettant l'inspiration et l'expiration par le nez et la bouche. Durant la phase inspiratoire (Figure 3), le patient peut inspirer librement par la bouche (8) ou par le nez (2). Le patient ne doit pas déclencher l'administration de l'aérosol. L'air inspiré et pénétrant dans les poumons (4) du patient est dépourvu d'aérosol. Après la phase inspiratoire, le patient réalise une apnée (Figure 4), et doit déclencher simultanément l'administration de l'aérosol (par pression manuelle (13) sur le dispositif par exemple). Les particules sont générées au niveau de la bouche (8) du patient et sont transportées de part leurs vitesses initiales jusque dans la cavité buccale (14) et le rhinopharynx (7). Le patient peut alors retirer le dispositif de sa bouche puis fermer ses lèvres tout en restant en apnée (Figure 5). Il réalise alors un mouvement de fermeture de la cavité buccale (14) d'avant en arrière (avec la mâchoire par exemple) afin de créer une modification de volume pour générer une surpression et faire déplacer l'aérosol vers le rhinopharynx (7). Le patient peut ensuite (ou simultanément) expirer (Figure 6) librement par le nez pour transporter les particules depuis le rhinopharynx (7) jusqu'aux narines (2) en passant par les fosses nasales (6). Dans ces conditions, l'aérosol ne passe pas en premier lieu par les narines et son efficacité de dépôt est augmentée.

A partir de ce principe, différentes configurations de mise en oeuvre de la méthode peuvent être réalisées.

L'embout buccal selon l'invention est étanche à l'air et pénètre au-delà des dents du patient selon une longueur maximale de 4 cm en constituant le moyen d'administration de l'aérosol à visée nasale, rhinopharyngée, ou nasosinusienne. Cette caractéristique dimensionnelle est spécifique de l'invention en égard des conditions d'application de l'aérosol. La longueur minima de pénétration de l'embout buccal au-delà des dents du patient est de 1 cm.

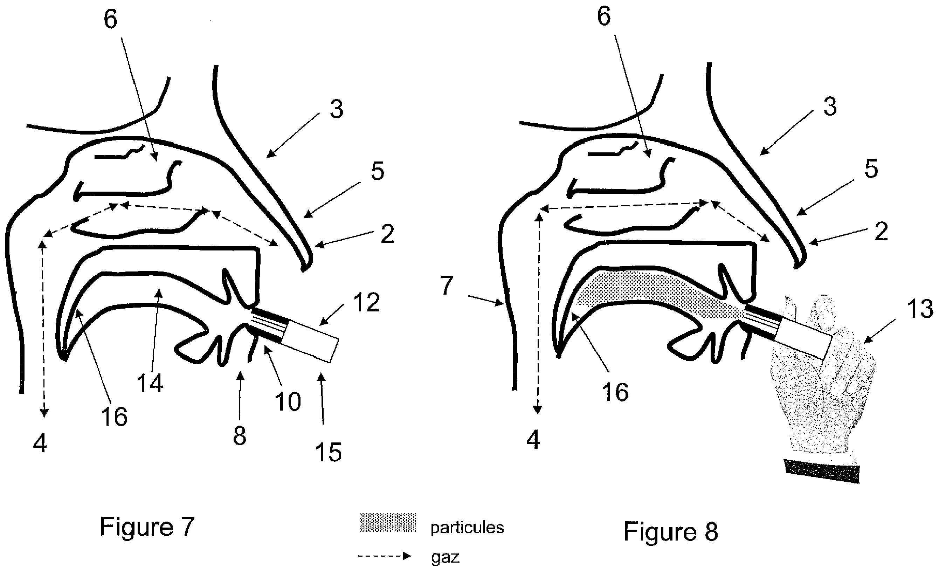

Une deuxième configuration du principe de la méthode de l'administration de l'aérosol avec un dispositif de type spray actionné manuellement (non conforme à l'invention) et ne permettant ni l'expiration ni l'inspiration par la bouche est illustrée aux figures 7, 8, 9 et 10.

Dans cette configuration (Figure 7), le dispositif (15) est un circuit étanche relié à la bouche (8) du patient comprenant un embout buccal (10) relié à un générateur de particules (12) fonctionnant sans addition de gaz.

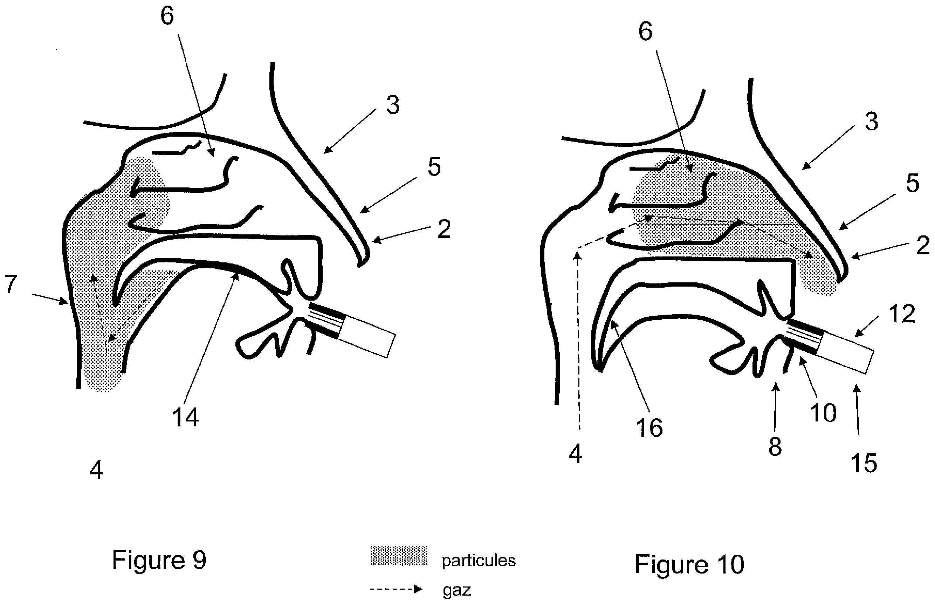

Ainsi, le système (15) est un circuit étanche ne permettant que l'inspiration et l'expiration par le nez. Durant la phase inspiratoire ou expiratoire nasale (Figure7), le voile du palais (16) ferme l'arrière cavité buccale (14), l'isolant des voies aériennes inférieures (4) et supérieures (3). Le patient peut alors déclencher (par pression manuelle (13) sur le dispositif par exemple) la génération de l'aérosol durant la phase d'inspiration (figure 8) sans que l'aérosol ne soit délivré dans les poumons (4) du patient. Le patient peut alors réaliser une apnée (Figure 9) puis un mouvement de fermeture de la cavité buccale (14) d'avant en arrière (avec la mâchoire par exemple) afin de créer une modification de volume pour générer une surpression et faire déplacer l'aérosol vers le rhinopharynx (7). Le patient peut ensuite expirer (Figure 10) par le nez pour transporter les particules depuis le rhinopharynx (7) jusqu'aux narines (2) en passant par les fosses nasales (6).

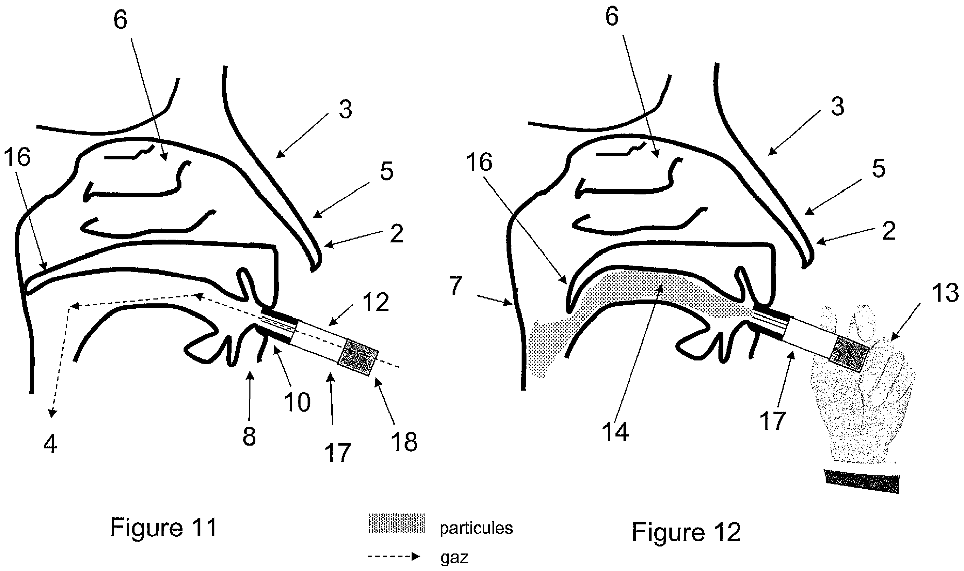

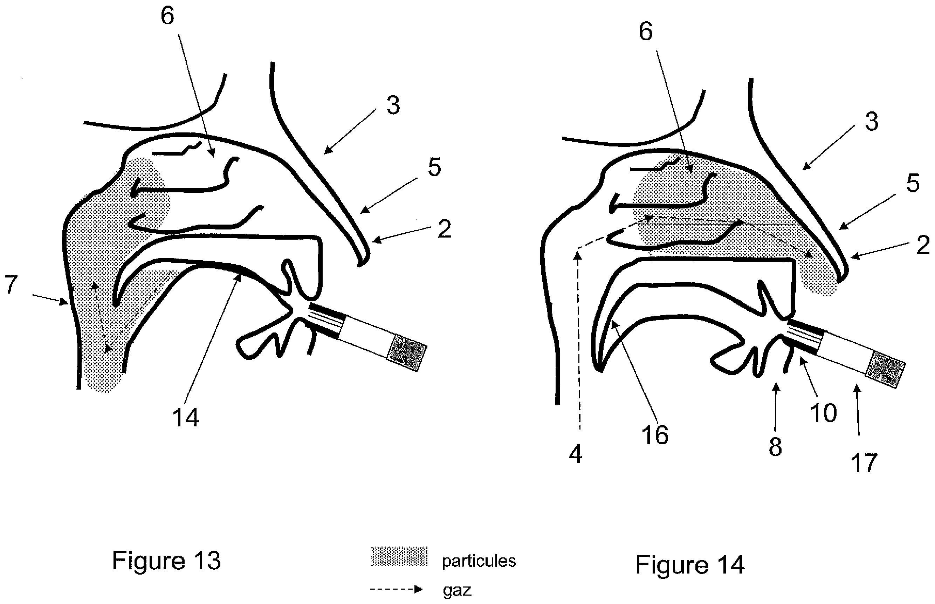

Une troisième configuration du principe de la méthode de l'administration de l'aérosol avec un dispositif de type spray valvé actionné manuellement et ne permettant pas l'expiration par la bouche est illustrée aux figures 11, 12, 13 et 14. Dans cette configuration (Figure 11), le dispositif (17) est un circuit relié à la bouche (8) du patient par l'intermédiaire d'un embout buccal (10). Ce dispositif (17) comprend un générateur de particules (12) fonctionnant sans addition de gaz et une valve inspiratoire (18). Ainsi, le système (17) est un circuit permettant l'inspiration par le nez et la bouche mais autorisant uniquement l'expiration par le nez. Durant la phase inspiratoire (Figure 11), le patient peut inspirer librement par la bouche (8) par l'intermédiaire de la valve (18) ou par le nez (2). Le patient ne doit pas déclencher l'administration de l'aérosol. L'air inspiré et pénétrant dans les poumons (4) du patient est dépourvu d'aérosol. Après la phase inspiratoire, le patient réalise une apnée (Figure 12), et doit déclencher simultanément l'administration de l'aérosol (par pression manuelle (13) sur le dispositif (17) par exemple). Les particules sont générées au niveau de la bouche (8) du patient et sont transportées de part leurs vitesses initiales jusque dans la cavité buccale (14) et le rhinopharynx (7). Il réalise alors un mouvement de fermeture de la cavité buccale (14) d'avant en arrière (avec la mâchoire par exemple) afin de créer une modification de volume pour générer une surpression et faire déplacer l'aérosol vers le rhinopharynx (7) (Figure 13). Le patient peut ensuite expirer (Figure 14) librement par le nez pour transporter les particules depuis le rhinopharynx (7) jusqu'aux narines (2) en passant par les fosses nasales (6).

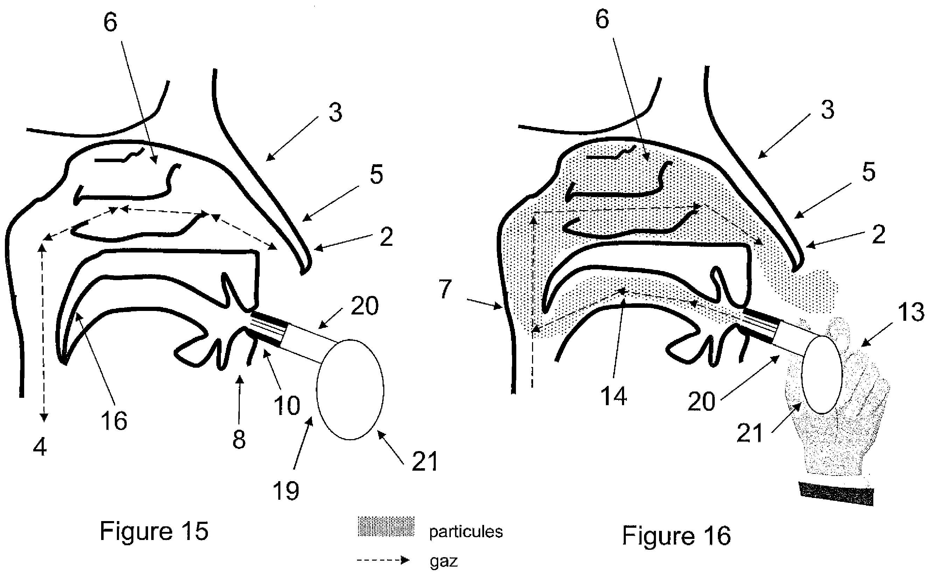

Une quatrième configuration du système est illustrée aux figures 15 et 16 et concerne le principe de fonctionnement du système (non conforme à l'invention) avec un générateur de type poudre et un réservoir de gaz externe pour la délivrance manuelle de l'aérosol. Dans cette configuration (Figure 15), le dispositif (19) est un circuit étanche relié à la bouche (8) du patient comprenant un embout buccal (10) relié à un générateur (20) de particules (poudre micronisée par exemple) fonctionnant à l'aide d'un réservoir de gaz externe (21) (poire déformable par exemple).

Ainsi, le système (19) est un circuit étanche permettant uniquement l'inspiration et l'expiration par le nez (2). Durant la phase inspiratoire (Figure 15), le patient ne peut qu'inspirer par le nez (2). L'aérosol n'est pas généré. L'air inspiré et pénétrant dans les poumons (4) du patient est dépourvu d'aérosol. Durant la phase expiratoire (Figure 16), le patient ne peut qu'expirer par le nez (2). Lors de son expiration nasale, le patient doit déclencher la génération de l'aérosol par pression manuelle (13) sur la poire (21) du dispositif (19). Les particules sont générées au niveau de la bouche (8) du patient et sont transportées par le gaz contenu dans le réservoir (21) (poire) jusqu'au rhinopharynx (7). L'air expiré par le patient va alors s'additionner au gaz vecteur provenant du dispositif (19) pour transporter les particules depuis le rhinopharynx (7) jusqu'aux narines (2) en passant par les fosses nasales (6).

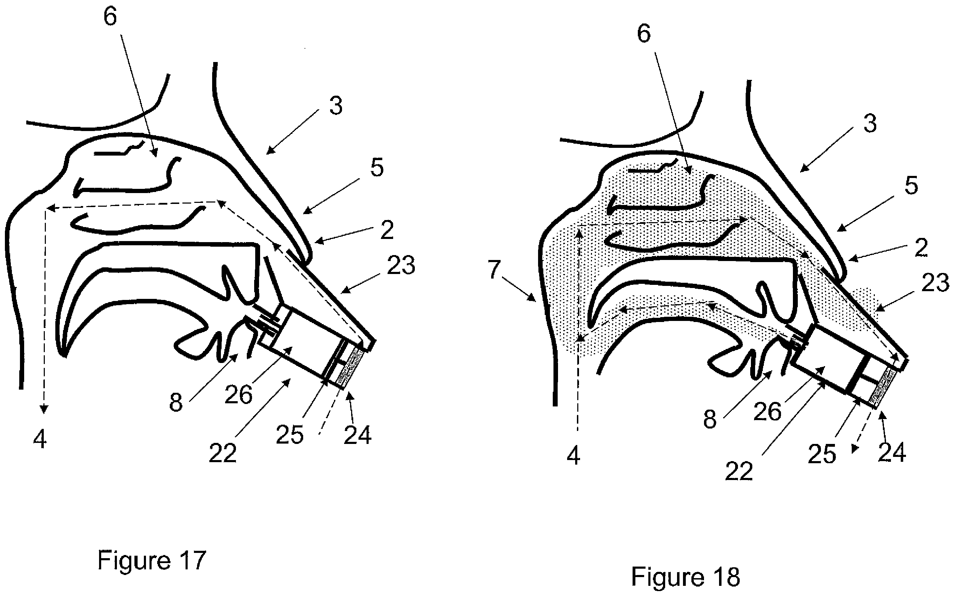

Les configurations illustrées aux figures 17 à 30 ci-après sont conformes à l'invention.

Une cinquième configuration du système est illustrée aux figures 17 et 18 et concerne le principe de fonctionnement du système selon l'invention avec un générateur de type flacon pressurisé et un réservoir de gaz externe pour la délivrance automatique de l'aérosol. Dans cette configuration, le dispositif (22) est connecté à la bouche (8) du patient et possède un embout narinaire (23) connecté aux narines (2) du patient. L'embout narinaire (23) est relié à un moyen mécanique (24) permettant le déclenchement d'un piston (25) durant la phase d'expiration nasale du patient. Le piston (25) permet le déclenchement de l'administration de l'aérosol par pression sur le générateur d'aérosol (26) comprenant un mélange de liquide et de gaz sous pression (flacon pressurisé par exemple). L'ensemble du système formant un ensemble étanche au niveau de la bouche du patient et permettant uniquement la respiration nasale. Durant la phase inspiratoire (Figure 17), le patient ne peut qu'inspirer par le nez (2). Le moyen mécanique (24) ne déclenche pas le piston (25): l'aérosol n'est pas généré. L'air inspiré et pénétrant dans les poumons (4) du patient est dépourvu d'aérosol. Durant la phase expiratoire (Figure 18), le patient ne peut qu'expirer par le nez (2). Le moyen mécanique (24) détecte une surpression, le piston (25) est déclenché, la pression sur le générateur d'aérosol (26) est exercée et l'aérosol est expulsé grâce au gaz sous pression contenu dans le générateur d'aérosol (26). L'aérosol est alors transporté par le gaz expulsé hors du générateur d'aérosol (26) depuis la bouche du patient (8) vers le rhinopharynx (7).

L'air expiré par le patient et provenant de ses poumons (4) est additionné au débit de gaz provenant du générateur d'aérosol (26). L'aérosol est alors dirigé depuis le rhinopharynx (7) vers les narines (2) puis est expulsé hors du patient.

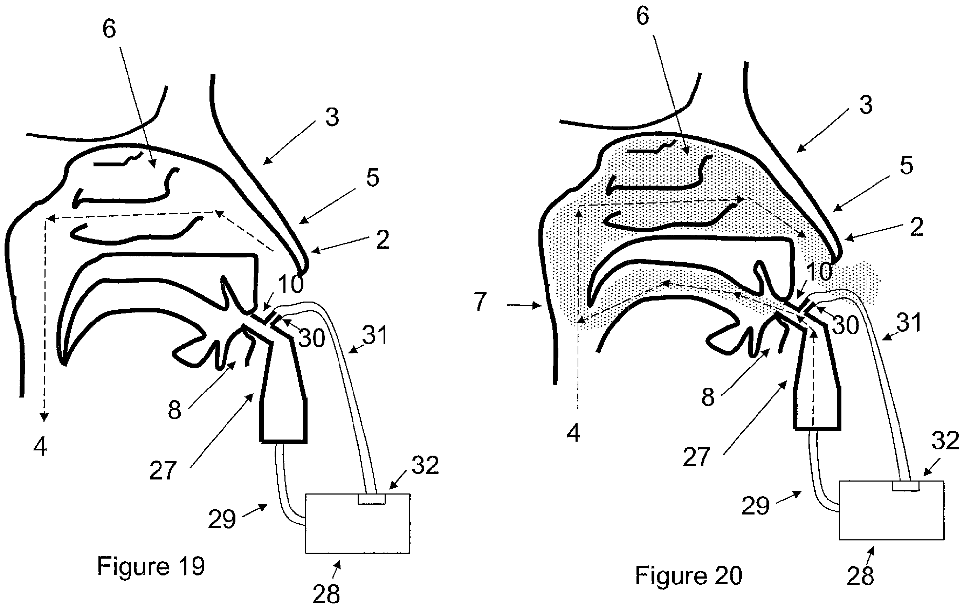

Une sixième configuration du système est illustrée aux figures 19 et 20 et concerne le principe de fonctionnement du système dans son application avec un nébuliseur pneumatique et un moyen automatique d'administration des particules. Dans cette configuration, le nébuliseur pneumatique (27) est connecté à la bouche (8) du patient et est alimenté par un compresseur d'air (28) par l'intermédiaire d'un tuyau (29). Le nébuliseur possède également une connexion (30) proche de l'embout buccal (10) destiné à recevoir un tuyau (31) lui-même relié au capteur de pression (32) contenu dans le compresseur (28). L'ensemble du système formant un ensemble étanche au niveau de la bouche du patient. Ainsi, durant la phase inspiratoire du patient (Figure 19), le patient ne peut qu'inspirer par le nez, le capteur de pression (32) ne détecte pas de surpression, l'aérosol n'est pas généré. Durant la phase expiratoire du patient (Figure 20), le patient ne peut qu'expirer par le nez. Le capteur de pression (32) détecte une surpression, le compresseur (28) fournit une pression au nébuliseur (27) et l'aérosol est généré. L'aérosol produit est alors transporté par l'air du compresseur (28) depuis la bouche du patient (8) vers le rhinopharynx (7). L'air expiré par le patient et provenant de ses poumons (4) est additionné au débit d'air provenant du compresseur (28). L'aérosol est alors dirigé depuis le rhinopharynx (7) vers les narines (2) puis est expulsé hors du patient.

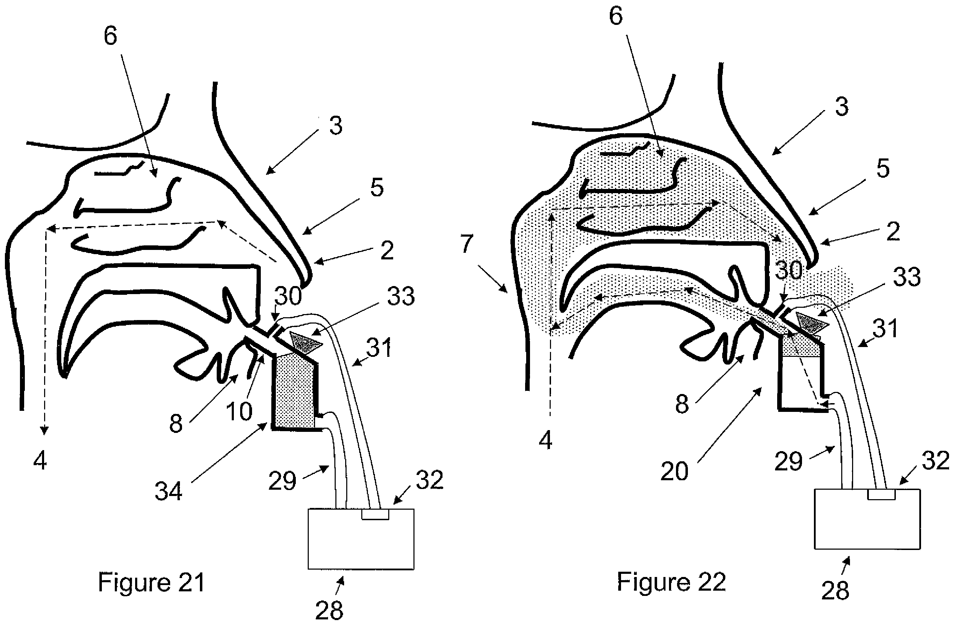

Une septième configuration du système est illustrée aux figures 21 et 22 et concerne le principe de fonctionnement du système dans son application avec un nébuliseur et une chambre de stockage associés à un moyen automatique d'administration des particules.
Dans cette configuration, le nébuliseur à tamis (33), ou ultrasonique, est associé à une chambre de stockage (34) connectée d'une part à la bouche (8) et d'autre part à une source d'air (28) (compresseur ou ventilateur par exemple). Le nébuliseur possède également une connexion (30) proche de l'embout buccal (10) destiné à recevoir un tuyau (31) lui-même relié au capteur de pression (32) contenu dans la source d'air (28). L'ensemble du système formant un ensemble étanche au niveau de la bouche du patient. Ainsi, durant la phase inspiratoire du patient (Figure 21), le patient ne peut qu'inspirer par le nez, le capteur de pression (32) ne détecte pas de surpression, l'air de la source (28) n'est pas généré. L'aérosol est produit en continu dans la chambre de stockage (34). Durant la phase expiratoire du patient (Figure 22), le patient ne peut qu'expirer par le nez. Le capteur de pression (32) détecte une surpression, la source (28) produit de l'air dans la chambre de stockage (34) et l'aérosol stocké est mis en mouvement. L'aérosol est alors transporté par l'air de la source (28) depuis la bouche du patient (8) vers l'arrière du rhinopharynx (7). L'air expiré par le patient et provenant de ses poumons (4) est additionné au débit d'air provenant de la source (28). L'aérosol est alors dirigé depuis le rhinopharynx (7) vers les narines (2) puis est expulsé hors du patient.

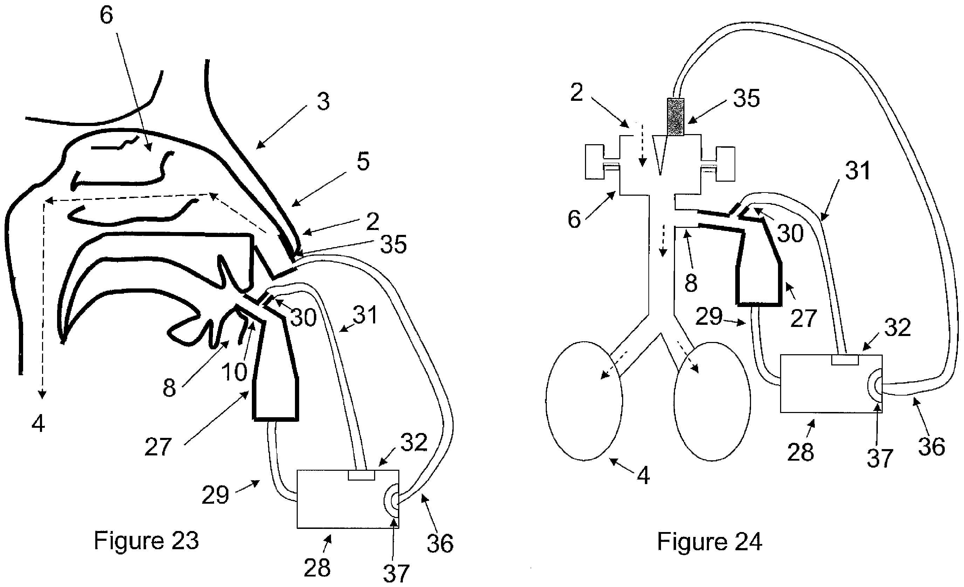

Une huitième configuration du système est illustrée aux figures 23, 24, 25 et 26 et concerne le principe de fonctionnement du système dans son application avec un nébuliseur pneumatique associé à une onde acoustique et à un moyen automatique d'administration des particules. Dans cette configuration, le nébuliseur pneumatique (27) connecté à la bouche (8) est alimenté par un compresseur d'air (28) par l'intermédiaire d'un tuyau (29).

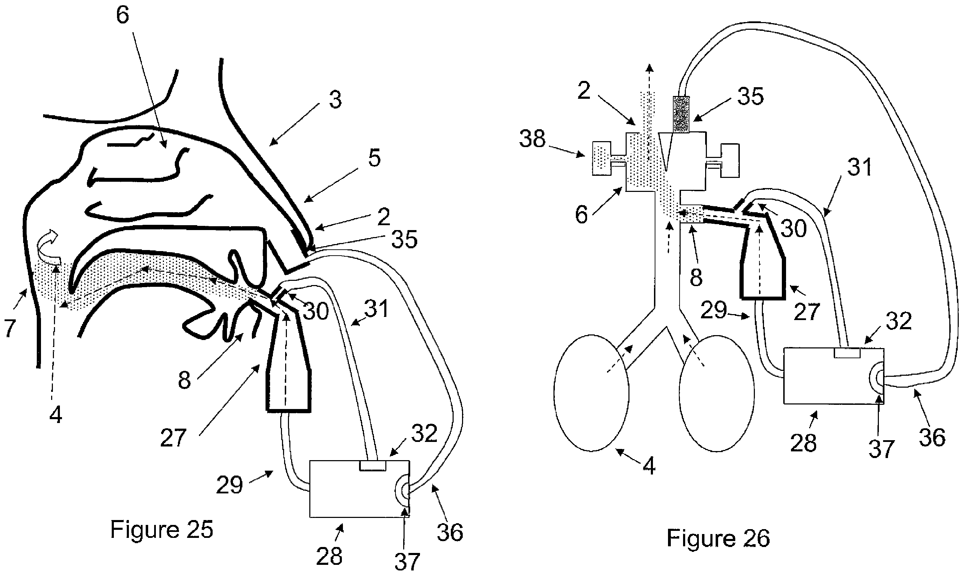

Le nébuliseur possède également une connexion (30) proche de l'embout buccal (10) destiné à recevoir un tuyau (31) lui-même relié au capteur de pression (32) contenu dans le compresseur (28). L'ensemble du système formant un ensemble étanche au niveau de la bouche du patient. Un embout nasal (35) est également connecté à une des deux narines (2). Un tuyau (36) destiné à transporter les ondes acoustiques relie la source d'ondes acoustiques (37) et l'embout nasal (35). Ainsi, durant la phase inspiratoire du patient (Figures 23 et 24), le patient ne peut qu'inspirer par une narine, le capteur de pression (32) ne détecte pas de surpression, l'aérosol n'est pas généré. Durant la phase expiratoire du patient (Figure 25 et 26), le patient ne peut expirer que par une seule narine. Le capteur de pression (32) détecte une surpression, le compresseur (28) fournit une pression au nébuliseur (27) et l'onde acoustique est produite depuis la source d'onde acoustique (37) à l'embout nasal (35). L'aérosol produit est alors transporté par l'air du compresseur (28) depuis la bouche du patient (8) vers le rhinopharynx (7). L'air expiré par le patient et provenant de ses poumons (4) est additionné au débit d'air provenant du compresseur (28). L'onde acoustique provenant de la première narine est transmise jusqu'au rhinopharynx et l'ensemble particule, gaz et onde est dirigé vers les fosses nasales (6). L'onde acoustique créant une pression acoustique va alors favoriser la pénétration de l'aérosol dans le sinus (38). L'aérosol est ensuite dirigé vers la narine ouverte puis est expulsé hors du patient.

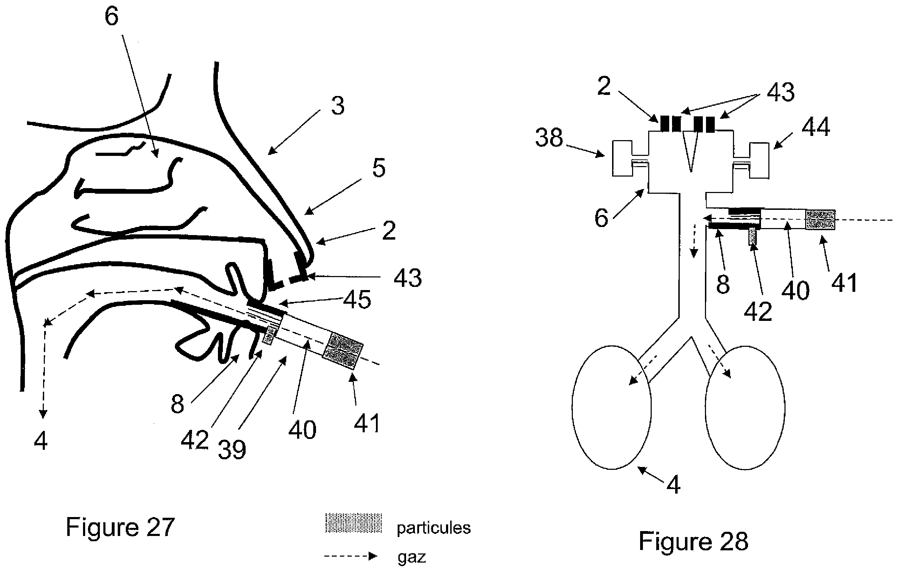

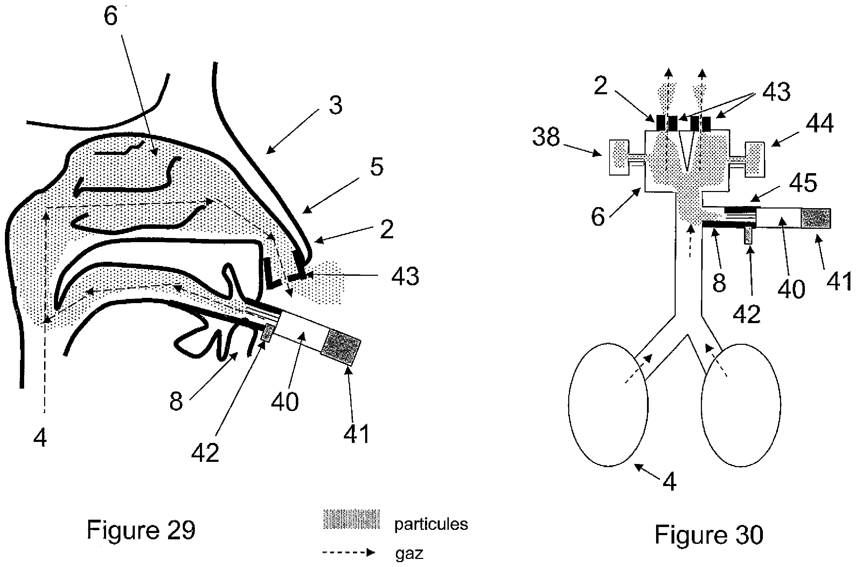

Une neuvième configuration du système est illustrée aux figures 27, 28, 29 et 30 et concerne le principe de fonctionnement du système dans son application avec un réservoir de gaz externe associé à un embout nasal créant une surpression dans les fosses nasales pour créer la pénétration de l'aérosol dans les sinus maxillaires. Le dispositif (39) est un circuit relié à la bouche (8) du patient par l'intermédiaire d'un embout buccal (45) pénétrant au-delà des dents du patient sur une longueur minimale de 1 cm (2cm par exemple) afin de garantir l'ouverture de la cavité buccale pour le passage de l'aérosol. Ce dispositif (39) comprend un générateur d'aérosol (40) incluant des particules et un gaz sous pression, une valve inspiratoire (41) ainsi qu'un capteur de pression (42) permettant le déclenchement de l'aérosol durant la phase expiratoire. Un embout nasal (43) muni d'un rétrécissement de section est également connecté aux deux narines (2). Ainsi, le système (39 et 43) est un circuit permettant et favorisant l'inspiration par la bouche mais autorisant uniquement l'expiration par le nez. Durant la phase inspiratoire (Figure 27 et 28), le patient inspire par la bouche (8) par l'intermédiaire de la valve (41). Le capteur (42) ne détecte pas de surpression : l'aérosol n'est pas généré. L'air inspiré et pénétrant dans les poumons (4) du patient est dépourvu d'aérosol. Durant la phase expiratoire (Figure 29 et 30), la valve (41) est fermée et le patient ne peut qu'expirer par le nez (2). Lors de son expiration nasale, le capteur de pression (42) détecte une surpression dans les voies aériennes et l'aérosol est généré par le générateur d'aérosol (40). Les particules sont générées au niveau de la bouche (8) du patient et sont transportées par le gaz propulseur du générateur d'aérosol (40) jusqu'au rhinopharynx (7). L'air expiré par le patient va alors transporter les particules depuis le rhinopharynx (7) jusqu'aux narines (2) en passant par les fosses nasales (6). Le rétrécissement de section de l'embout nasal (43) créé une surpression au niveau des voies aériennes supérieures et favorise la pénétration de l'aérosol dans les sinus (38 et 44). L'aérosol est ensuite dirigé vers les narines puis est expulsé hors du patient.

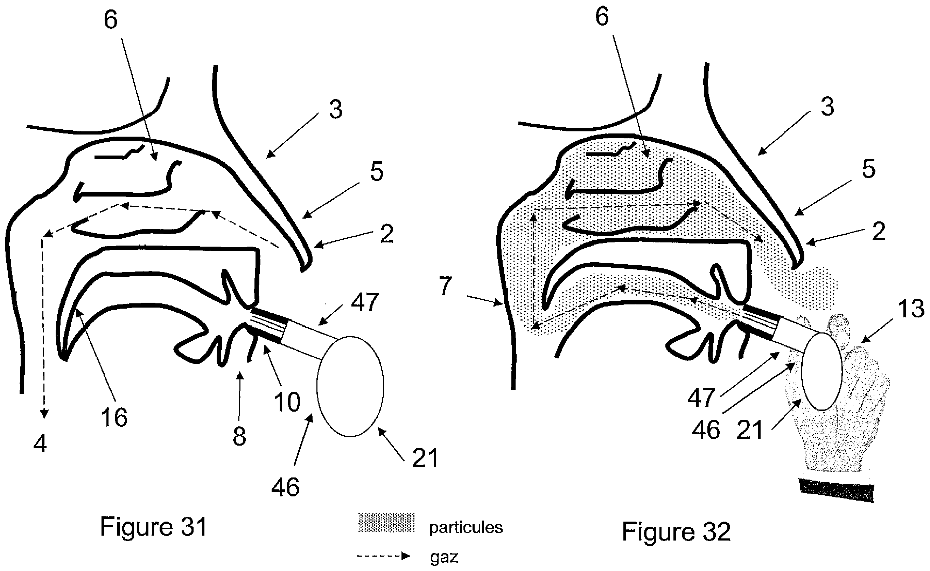

Une dixième configuration du système est illustrée aux figures 31, 32, 33 et 34 et concerne le principe de fonctionnement du système (non conforme à l'invention) avec un générateur de poudre et un réservoir de gaz externe pour la délivrance manuelle de l'aérosol durant la première partie de la pause inspiratoire. Dans cette configuration (Figure 31), le dispositif (46) est un circuit étanche relié à la bouche (8) du patient comprenant un embout buccal (10) relié à un générateur (47) de particules (poudre micronisée par exemple) fonctionnant à l'aide d'un réservoir de gaz externe (21) (poire déformable par exemple) administrant les particules de poudre uniquement durant la première période de la génération de gaz par déformation de la poire.

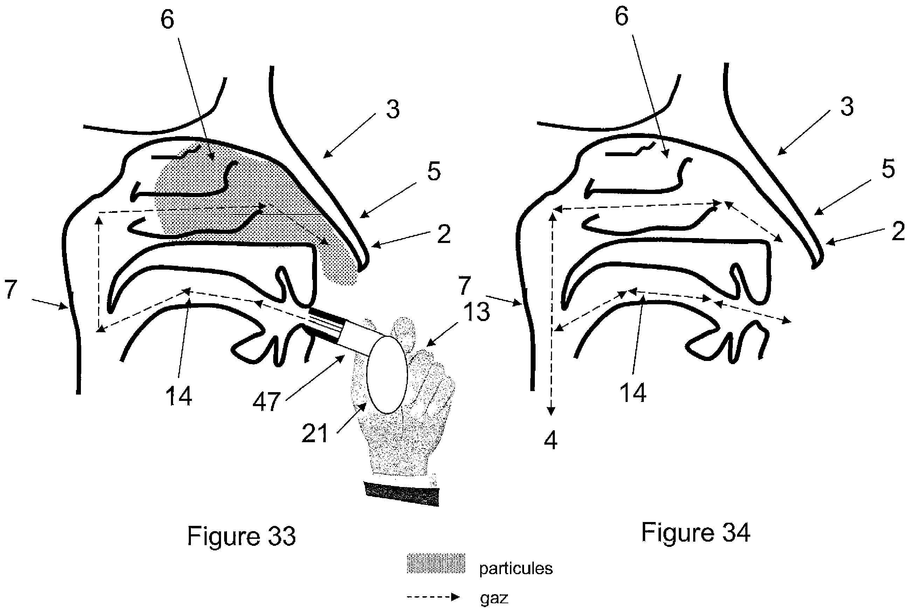

Ainsi, le système (46) est un circuit étanche permettant uniquement l'inspiration et l'expiration par le nez (2). Durant la phase inspiratoire (Figure 31), le patient ne peut qu'inspirer par le nez (2). L'aérosol n'est pas généré. L'air inspiré et pénétrant dans les poumons (4) du patient est dépourvu d'aérosol. Le patient effectue alors une pause respiratoire (Figure 32) et déclenche simultanément la génération de l'aérosol par pression manuelle (13) sur la poire (21) du dispositif (46). Durant la première partie de pause inspiratoire, les particules sont générées au niveau de la bouche (8) du patient et sont transportées par le gaz contenu dans le réservoir (21) (poire) jusqu'aux narines (7). Durant la deuxième partie de pause respiratoire (Figure 33), le gaz produit par la poire (21) et généré au niveau de la bouche (8) du patient est dépourvu de particules. Le gaz dépourvu de particules va remplir la bouche, le rhinopharynx, les fosses nasales puis les narines, assurant ainsi le rinçage de cette zone par du gaz dépourvu de particules en suspension. Le patient peut ensuite expirer ou inspirer librement par le nez ou la bouche un gaz dépourvu de particules (Figure 34).

Dans cette configuration, l'aérosol n'est pas généré dans les poumons du patient (4) et la vitesse des particules produites peut être contrôlée par le débit du générateur de gaz (21).

Dans les configurations précitées, les formes des circuits peuvent varier en forme, les figures ayant été décrites et citées à titre d'exemple. L'utilisation d'un dispositif ne comprenant pas de système de délivrance de gaz pour véhiculer les particules de médicament demande une participation active du patient. Après la délivrance du médicament dans la cavité buccale, le patient doit réaliser une modification de son volume interne buccal (déglutition ou fermeture de la mâchoire) pour déplacer les particules depuis la cavité buccale jusqu'au du rhinopharynx puis doit expirer par le nez. Ce principe de méthode d'administration d'aérosol à visée rhinopharyngée, nasosinusienne ou nasale est décrit dans la première, deuxième et troisième configuration. L'utilisation d'un dispositif comprenant un système de délivrance de gaz pour les particules pour véhiculer le médicament demande une participation moins active du patient. Dans ce cas, le patient doit uniquement synchroniser la délivrance manuelle de l'aérosol à son expiration nasale ou sa pause respiratoire. Ce principe de fonctionnement de système d'aérosol à visée rhinopharyngée, nasosinusienne ou nasale est décrit dans la quatrième et dixième configuration. Ce système possède l'avantage d'être très simple d'utilisation et pourrait être comparé au principe des aérosols doseurs (pMDI) à visée pulmonaire demandant à la fois une inspiration buccale et un réflexe de déclenchement manuel de l'administration de l'aérosol lors de sa phase d'inspiration. Pour limiter les erreurs éventuelles de l'utilisation du dispositif dans sa forme la plus simple, l'utilisation d'une valve inspiratoire (configuration 9) ou d'un dispositif étanche au niveau de la bouche du patient (configurations 4, 5, 6, 7, 8 et 10) sont utilisés pour ne pas autoriser l'expiration buccale lors des phases d'administration de l'aérosol. De même l'utilisation d'un moyen automatique pour l'administration de l'aérosol durant la phase non inspiratoire (phase expiratoire ou phase de pause respiratoire) permet de s'affranchir du problème de réflexe manuel du patient (configurations 5, 6, 7, 8 et 9). En conséquence, le moyen permettant l'administration des particules dans la bouche est un moyen automatique, à la différence d'un moyen non automatique (par exemple : moyen manuel activé par le patient lui-même ou une autre personne). Le type du générateur d'aérosol peut varier. Un générateur d'aérosol de type nébuliseur pneumatique, ultrasonique ou à tamis peut être utilisé. De même, tout autre générateur d'aérosol liquide ou solide peut être utilisé (Aérosol doseur de poudre ou de liquide, Dry Powder Inhaler ou Metered Dose Inhaler). Un système à piston suivi d'un injecteur (spray, Microsprayer...) ou encore un système de pré chargement de poudre dans un tuyau ou dans une gélule peut être également utilisé. Un embout nasal supplémentaire peut également être utilisé pour transmettre une onde ou créer une surpression. L'administration de l'aérosol peut être réalisée durant la totalité ou une partie des phases d'expirations nasales ou des phases de pauses respiratoires.

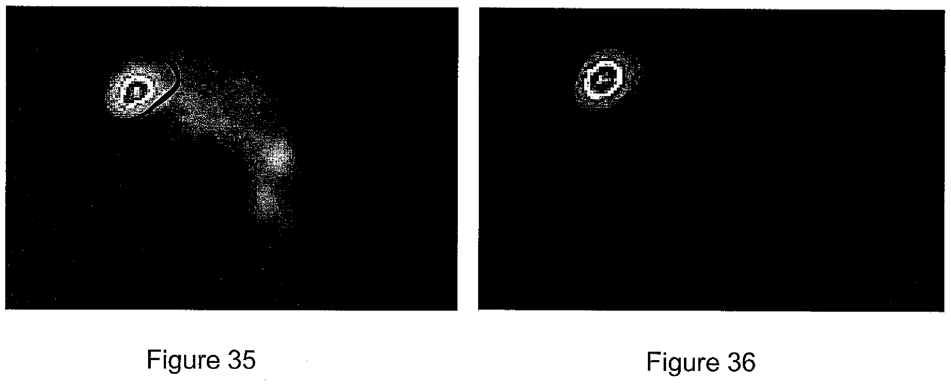

Suivant l'une quelconque des configurations décrites précédemment, la solution apparaît extrêmement avantageuse, car selon les tests effectués, il a été mesuré que la dose d'aérosol radioactif déposé dans les fosses nasales est augmentée de façon significative en comparaison de la dose d'aérosol radioactif déposé dans les narines (Tableau 2) (Figures 35 et 36).

**Tableau 2 : Ratio de l'aérosol déposé dans les fosses nasales par l'aérosol déposé dans les narines. Etude de la distribution de l'aérosol radioactif dans la sphère ORL de sujets sains.**

| | **Nébuliseur Atomisor NL11 Administration nasale** | **Invention Administration buccale** |
|---|---|---|
| **1** | **0.01** | **1.43** |
| **2** | **0.1** | **1.35** |

## Revendications

1. Dispositif (27 ; 33 ; 39 ; 46) d'administration d'aérosol constitué :
- d'un générateur (26 ; 27 ; 33 ; 40 ; 47) de particules dont la taille est comprise entre 10 nm et 200 µm,
- d'une source de gaz ou pression pour véhiculer les particules, et
- d'un embout buccal (10 ; 45) ou masque buccal étanche à l'air, apte à pénétrer au-delà des dents du patient selon une longueur de 4 cm maximale, et permettant l'administration de l'aérosol par la bouche durant la phase d'expiration nasale ou durant la phase de pause respiratoire précédent l'expiration nasale,
**caractérisé en ce que** le dispositif comprend un moyen (24 ; 32 ; 42) électrique, pneumatique ou mécanique de déclenchement automatique de la génération de l'aérosol durant la phase d'expiration nasale ou durant la phase de pause respiratoire précédent l'expiration nasale, apte à détecter des surpressions ou des débits d'air, et à commander la génération de particules, **en ce que** l'embout buccal (10 ; 45) constitue ainsi un moyen d'administration de l'aérosol à visée nasale, rhinopharyngée ou nasosinusienne durant l'administration de l'aérosol et permettant d'acheminer l'aérosol successivement dans la bouche, le rhinopharynx puis les fosses nasales et les sinus jusqu'à son échappement par au moins une narine du patient,
et **en ce que** le dispositif n'autorise pas l'expiration buccale lors des phases d'administration de l'aérosol, les particules d'aérosols n'étant pas dirigées vers les poumons.

2. Dispositif selon la revendication 1, **caractérisé en ce que** l'embout buccal est apte à pénétrer au-delà des dents selon une longueur minimale de 1 cm.

3. Dispositif (22) selon la revendication 1, **caractérisé en ce qu'**il comprend en outre un embout narinaire (23) connecté aux narines du patient, et **en ce que** ledit embout narinaire (23) est relié à un moyen mécanique (24) permettant le déclenchement d'un piston. (25) durant la phase d'expiration nasale du patient.

4. Dispositif (27) selon la revendication 1, **caractérisé en ce qu'**il comprend un nébuliseur pneumatique (27) relié à la bouche du patient et alimenté par un compresseur d'air (28) par l'intermédiaire d'un tuyau (29), ledit nébuliseur étant connecté à l'embout buccal (10) recevant un tuyau (31) relié à un capteur de pression (32) contenu dans le compresseur (28), et **en ce que** le nébuliseur est déclenché lors de la phase d'expiration nasale du patient, détectée par le capteur de pression (32) relié de manière étanche à la cavité buccale du patient.

5. Dispositif selon la revendication 1, **caractérisé en ce qu'**il comprend un nébuliseur (33) et une chambre de stockage (34) connectée à la bouche (8) et à une source de gaz (28), ledit nébuliseur étant connecté à l'embout buccal (10) recevant un tuyau (31) lié à un capteur de pression (32) contenu dans la source de gaz (28), et **en ce que** le nébuliseur est déclenché lors de la phase d'expiration nasale du patient, détectée par le capteur de pression (32) relié de manière étanche à la cavité buccale du patient.

6. Dispositif (27) selon la revendication 1, **caractérisé en ce qu'**il comprend un nébuliseur pneumatique (27) associé à une onde acoustique et à des moyens d'administration des particules, et **en ce que** le dit nébuliseur est connecté à la bouche et alimenté par un compresseur d'air (28) par l'intermédiaire d'un tuyau (29), le dit nébuliseur possédant une connexion (30) proche de l'embout buccal (10) destiné à recevoir un tuyau (31), lui-même relié à un capteur de pression (32) contenu dans le dit compresseur (28), l'ensemble étant étanche au niveau de la bouche du patient, et **en ce qu'**il comprend un embout nasal (35) connecté à une des deux narines (2) du patient, un tuyau (36) destiné à transporter les ondes acoustiques reliant la source d'onde acoustique (37) et l'embout nasal (35).

7. Dispositif (19 ; 46) selon la revendication 1, **caractérisé en ce que** l'embout buccal (10) est relié à un générateur (20 ; 47) de particules de poudres fonctionnant à l'aide d'un réservoir de gaz externe (21).

8. Dispositif (27) selon la revendication 1, **caractérisé en ce qu'**il comprend un nébuliseur pneumatique (27) connecté à la bouche du patient et alimenté par un compresseur d'air (28) par l'intermédiaire d'un tuyau (29), le dit nébuliseur possédant une connexion (30) proche de l'embout buccal (10) destiné à recevoir un tuyau (31) relié à un capteur de pression (32) contenu dans le dit compresseur (28), et **en ce que** l'ensemble est étanche au niveau de la bouche du patient.

9. Dispositif (33) selon la revendication 1, **caractérisé en ce qu'**il comprend un nébuliseur associé à une chambre de stockage (34) connectée d'une part à la bouche (8) et d'autre part à une source d'air, et **en ce que** le dit nébuliseur possède une connexion (30) proche de l'embout buccal (10) destiné à recevoir un tuyau (31) relié à un capteur de pression (32) contenu dans un compresseur d'air (28), l'ensemble étant étanche au niveau de la bouche du patient.

10. Dispositif (39) selon la revendication 1, **caractérisé en ce qu'**il comprend un générateur d'aérosol (40) incluant des particules et un gaz sous pression, une valve inspiratoire (41) et un capteur de pression (42) permettant le déclenchement de l'aérosol durant la phase expiratoire, et **en ce qu'**il comprend un embout nasal (43) muni d'un rétrécissement de section et connecté aux deux narines (2).

11. Dispositif (46) selon la revendication 1, **caractérisé en ce qu'**il définit un circuit étanche relié à la bouche du patient comprenant un embout buccal (10) relié à un générateur (47) de particules de poudre fonctionnant à l'aide d'un réservoir de gaz externe administrant les particules de poudre uniquement durant la première période de la génération de gaz par la déformation du dit réservoir (21).

## Patentansprüche

1. Vorrichtung (27 ; 33 ; 39 ; 46) zur Aerosolverabreichung, bestehend aus:
- einem Erzeugungsgerät (26 ; 27 ; 33 ; 40 ; 47) für Partikel, deren Größe im Bereich von 10 nm bis 200 µm liegt,
- einer Gas- oder Druckquelle, um die Partikel zu befördern, und
- einem luftdichten Mundstück (10 ; 45) oder einer solchen Mundmaske, das/die dazu befähigt ist, mit einer Länge von höchstens 4 cm über die Zähne des Patienten hinaus einzudringen, wobei es/sie ermöglicht, das Aerosol während der Phase der Nasenausatmung oder während der Phase der Atempause, welche der Nasenausatmung vorangeht, durch den Mund zu verabreichen,
**dadurch gekennzeichnet, dass** die Vorrichtung ein Mittel (24 ; 32 ; 42) elektrischer, pneumatischer oder mechanischer Art umfasst, das zur automatischen Auslösung der Erzeugung des Aerosols während der Phase der Nasenausatmung oder während der Phase der Atempause, welche der Nasenausatmung vorangeht, bestimmt ist, wobei es dazu befähigt ist, Überdrücke oder Luftströme zu detektieren und die Erzeugung von Partikeln zu steuern, sowie dadurch, dass das Mundstück (10 ; 45) somit während der Verabreichung des Aerosols ein Mittel zur Verabreichung des Aerosols in die Nase, den Nasen-Rachenraum oder die Nasennebenhöhlen darstellt, wobei mittels desselben das Aerosol nacheinander in den Mund, den Nasen-Rachenraum, die Nasenhöhle und die Nebenhöhlen geleitet werden kann, woraufhin es durch mindestens ein Nasenloch des Patienten austritt,
und dadurch, dass die Vorrichtung während der Phasen der Verabreichung des Aerosols keinerlei Mundausatmung gestattet, sodass die Aerosolpartikel nicht der Lunge zugeführt werden.

2. Vorrichtung nach Anspruch 1, **dadurch gekennzeichnet, dass** das Mundstück dazu befähigt ist, mit einer Länge von mindestens 1 cm über die Zähne hinaus einzudringen.

3. Vorrichtung (22) nach Anspruch 1, **dadurch gekennzeichnet, dass** sie darüber hinaus ein Nasenlochstück (23) umfasst, welches an die Nasenlöcher des Patienten angeschlossen ist, sowie dadurch, dass das Nasenlochstück (23) mit einem mechanischen Mittel (24) verbunden ist, welches das Auslösen eines Kolbens (25) während der Phase der Nasenausatmung des Patienten ermöglicht.

4. Vorrichtung (27) nach Anspruch 1, **dadurch gekennzeichnet, dass** sie ein pneumatisches Vernebelungsgerät (27) umfasst, das mit dem Mund des Patienten verbunden ist und über einen Schlauch (29) von einem Luftkompressor (28) gespeist wird, wobei das Vernebelungsgerät an das Mundstück (10) angeschlossen ist, welches einen Schlauch (31) aufnimmt, der mit einem Drucksensor (32) verbunden ist, welcher in dem Kompressor (28) enthalten ist, sowie dadurch, dass das Vernebelungsgerät in der Phase der Nasenausatmung des Patienten ausgelöst wird, wobei diese von dem Drucksensor (32) detektiert wird, der abdichtend mit der Mundhöhle des Patienten verbunden ist.

5. Vorrichtung nach Anspruch 1, **dadurch gekennzeichnet, dass** sie ein Vernebelungsgerät (33) und eine Speicherkammer (34) umfasst, wobei letztere an den Mund (8) und an eine Gasquelle (28) angeschlossen ist, wobei das Vernebelungsgerät an das Mundstück (10) angeschlossen ist, welches einen Schlauch (31) aufnimmt, der mit einem Drucksensor (32) verbunden ist, welcher in der Gasquelle (28) enthalten ist, sowie dadurch, dass das Vernebelungsgerät in der Phase der Nasenausatmung des Patienten ausgelöst wird, wobei diese von dem Drucksensor (32) detektiert wird, der abdichtend mit der Mundhöhle des Patienten verbunden ist.

6. Vorrichtung (27) nach Anspruch 1, **dadurch gekennzeichnet, dass** sie ein pneumatisches Vernebelungsgerät (27) in Verbindung mit einer Schallwelle und mit Mitteln zur Verabreichung von Partikeln umfasst, sowie dadurch, dass das Vernebelungsgerät an den Mund angeschlossen ist und über einen Schlauch (29) von einem Luftkompressor (28) gespeist wird, wobei das Vernebelungsgerät eine Anschlussstelle (30) besitzt, die sich in der Nähe des Mundstücks (10) befindet und dazu bestimmt ist, einen Schlauch (31) aufzunehmen, welcher seinerseits mit einem Drucksensor (32) verbunden ist, der in dem Kompressor (28) enthalten ist, wobei die Gesamteinheit im Bereich des Mundes des Patienten abgedichtet ist, und dadurch, dass sie ein Nasenstück (35) umfasst, dass an eines der beiden Nasenlöcher (2) des Patienten angeschlossen ist, wobei ein Schlauch (36), der zum Transport der Schallwellen bestimmt ist, die Schallwellenquelle (37) mit dem Nasenstück (35) verbindet.

7. Vorrichtung (19 ; 46) nach Anspruch 1, **dadurch gekennzeichnet, dass** das Mundstück (10) mit einem Erzeugungsgerät (20 ; 47) für Pulverpartikel verbunden ist, welches mittels eines externen Gasbehälters (21) betrieben wird.

8. Vorrichtung (27) nach Anspruch 1, **dadurch gekennzeichnet, dass** sie ein pneumatisches Vernebelungsgerät (27) umfasst, das mit dem Mund des Patienten verbunden ist und über einen Schlauch (29) von einem Luftkompressor (28) gespeist wird, wobei das Vernebelungsgerät eine Anschlussstelle (30) besitzt, die sich in der Nähe des Mundstücks (10) befindet und dazu bestimmt ist, einen Schlauch (31) aufzunehmen, welcher mit einem Drucksensor (32) verbunden ist, der in dem Kompressor (28) enthalten ist, sowie dadurch, dass die Gesamteinheit im Bereich des Mundes des Patienten abgedichtet ist.

9. Vorrichtung (33) nach Anspruch 1, **dadurch gekennzeichnet, dass** sie ein Vernebelungsgerät in Verbindung mit einer Speicherkammer (34) umfasst, wobei letztere einerseits an den Mund (8) und andererseits an eine Luftquelle angeschlossen ist, sowie dadurch, dass das Vernebelungsgerät eine Anschlussstelle (30) besitzt, die sich in der Nähe des Mundstücks (10) befindet und dazu bestimmt ist, einen Schlauch (31) aufzunehmen, welcher mit einem Drucksensor (32) verbunden ist, der in einem Luftkompressor (28) enthalten ist, wobei die Gesamteinheit im Bereich des Mundes des Patienten abgedichtet ist.

10. Vorrichtung (39) nach Anspruch 1, **dadurch gekennzeichnet, dass** sie ein Aerosolerzeugungsgerät (40) umfasst, welches Partikel und ein unter Druck stehendes Gas, ein Einatmungsventil (41) sowie einen Drucksensor (42) beinhaltet, mittels dessen das Aerosol während der Ausatemphase ausgelöst werden kann, sowie dadurch, dass sie ein Nasenstück (43) umfasst, welches mit einer Querschnittsverengung versehen ist und an die beiden Nasenlöcher (2) angeschlossen ist.

11. Vorrichtung (46) nach Anspruch 1, **dadurch gekennzeichnet, dass** sie ein abgedichtetes Kreislaufsystem beschreibt, das mit dem Mund des Patienten verbunden ist, wobei es ein Mundstück (10) umfasst, welches mit einem Erzeugungsgerät (47) für Pulverpartikel verbunden ist, das mittels eines externen Gasbehälters betrieben wird, wobei es die Pulverpartikel ausschließlich in dem Zeitraum verabreicht, während dessen durch Verformung des Behälters (21) Gas erzeugt wird.

## Claims

1. Aerosol administration device (27 ; 33 ; 39 ; 46) consisting of:
- a generator (26 ; 27 ; 33 ; 40 ; 47) of particles of which the size is between 10nm and 200µm,
- a source of gas or pressure for conveying the particles, and
- a mouthpiece (10 ; 45) or airtight oral mask, capable of penetrating beyond the patient's teeth to a maximum length of 4cm, and allowing the administration of the aerosol by the mouth during the nasal expiration phase or during the respiratory pause phase preceding nasal expiration,
**characterised in that** the device comprises electric, pneumatic or mechanical means (24; 32; 42) of automatic triggering of the generation of the aerosol during the nasal expiration phase or during the respiratory pause phase preceding nasal expiration, capable of detecting overpressure or airflow and commanding particles generation,
**in that** the mouthpiece (10 ; 45) thus constitutes means of administration of aerosol to the nasal cavities, rhinopharynx or paranasal sinuses during the administration of the aerosol and enabling the aerosol to be successively conveyed to the mouth, the rhinopharynx then the nasal fossae and sinuses, to escape through at least one of the patient's nostrils,
and **in that** the device does not allow oral expiration during the aerosol administration phases, the aerosol particles not being directed to the lungs.

2. Device according to claim 1, **characterised in that** the mouthpiece penetrates beyond the teeth by a minimum length of 1cm.

3. Device (22) according to claim 1, **characterised in that** it further comprises a nosepiece (23) connected to the patient's nostrils, and **in that** said nosepiece (23) is connected to a mechanical means (24) allowing the triggering of a piston (25) during the patient's nasal expiration phase.

4. Device (27) according to claim 1, **characterised in that** it comprises a pneumatic nebulizer (27) connected to the patient's mouth and supplied by an air compressor (28) via a tube (29), said nebulizer being connected to the mouthpiece receiving a tube (31) connected to a pressure sensor (32) contained in the compressor (28), and **in that** the nebuliser is triggered during the patient's nasal expiration phase, detected by the pressure sensor (32) connected in a sealed manner to the patient's oral cavity.

5. Device according to claim 1, **characterised in that** it includes a nebulizer (33) and a storage chamber (34) connected to the mouth (8) and to a source of gas (28), said nebulizer being connected to the mouthpiece (10) receiving a tube (31) connected to a pressure sensor (32) contained in the gas source (28), and **in that** the nebulizer is triggered during the patient's nasal expiration phase, detected by the pressure sensor (32) connected in a sealed manner to the patient's oral cavity.

6. Device (27) according to claim 1, **characterised in that** it comprises a pneumatic nebulizer (27) associated with an acoustic wave and particle administration means, and **in that** said nebulizer is connected to the mouth and supplied by an air compressor (28) via a tube (29), said nebulizer having a connection (30) near the mouthpiece (10) designed to receive a tube (31), itself connected to a pressure sensor (32) contained in said compressor (28), the assembly being sealed at the patient's mouth, and **in that** it comprises a nosepiece (35) connected to one of the patient's two nostrils (2), a tube (36) designed to convey the acoustic waves connecting the acoustic-wave source (37) and the nosepiece (35).

7. Device (19 ; 46) according to claim 1, **characterised in that** the mouthpiece (10) is connected to a generator (20 ; 47) of powder particles operating with the aid of an external gas reservoir (21).

8. Device (27) according to claim 1, **characterised in that** it comprises a pneumatic nebuliser (27) connected to the patient's mouth and supplied by an air compressor (28) via a tube (29), said nebulizer having a connection (30) near the mouthpiece (10) designed to receive a tube (31) connected to a pressure sensor (32) contained in said compressor (28), and **in that** the assembly is sealed at the patient's mouth.

9. Device (33) according to claim 1, **characterised in that** it comprises a nebulizer associated with a storage chamber (34) connected at one end to the mouth (8) and at the other end to an air source, and **in that** said nebulizer has a connection (30) near the mouthpiece (10) designed to receive a tube (31) connected to a pressure sensor (32) contained in the air compressor (28), the assembly being sealed at the patient's mouth.

10. Device (39) according to claim 1, **characterised in that** it comprises an aerosol generator (40) including particles and pressurized gas, an inspiratory valve (41) and a pressure sensor (42) allowing triggering of the aerosol during the expiratory phase, and **in that** it comprises a nosepiece (43) having a narrow section and connected to both nostrils (2).

11. Device (46) according to claim 1, **characterised in that** it defines a sealed circuit connected to the patient's mouth comprising a mouthpiece (10) connected to a generator (47) of powder particles operating with the aid of an external gas reservoir administering particles of powder only during the first period of the generation of gas by the deformation of said reservoir (21).
